# EUROPEAN PATENT SPECIFICATION

(11) **EP 2 059 202 B1**
(45) Date of publication and mention of the grant of the patent: **14.08.2013**
(21) Application number: 07841361.4
(22) Date of filing: 24.08.2007
(51) Int. Cl.: A61F 9/008, A61B 18/20

(54) **SYSTEM FOR MARKING CORNEAL TISSUE IN A TRANSPLANT PROCEDURE**
SYSTEM FÜR HORNHAUTGEWEBEMARKIERUNG BEI EINEM TRANSPLANTATIONSVERFAHREN
SYSTEME DE MARQUAGE DU TISSU CORNEEN DANS UNE PROCEDURE DE TRANSPLANTATION

(30) Priority: 05.09.2006 US 469901
(43) Date of publication of application: 20.05.2009
(73) Proprietor: AMO Development, LLC, Santa Ana, CA 92705 (US)
(72) Inventor: KURTZ, Ronald M., Irvine, California 92603 (US); SARAYBA, Melvin A., Ladera Ranch, California 92694 (US); STEINERT, Roger, Laguna Beach, California 92651 (US)
(74) Representative: Clark, Jane Anne
(86) International application number: PCT/US2007/076809
(87) International publication number: WO 2008/030718

(56) References cited:
- EP-A1- 0 541 316
- US-A- 5 312 428
- US-A- 5 549 632
- US-A- 5 571 124
- US-A- 5 752 967
- US-A- 6 110 166
- US-B1- 6 325 792

## Description

### BACKGROUND OF THE INVENTION

The field of the present invention is systems for transplanting corneas.

A variety of techniques presently exist for performing both full thickness corneal transplants and lamellar corneal transplants. The tools used for the different techniques range from the traditional trephine to the more advanced laser surgical systems. Regardless of the technique or tool used for either type of transplant procedure, the overarching goal remains to provide the recipient with new corneal tissue for the best possible post-operative optical quality. One of the most common post-operative complications is induced astigmatism, and improvements on current techniques are needed to minimize, if not eliminate, this common problem.

US 6,325,792 describes an ophthalmic surgical laser and method in which low energy ultra-short pulsed laser radiation is applied to the patient's eye in one of a number of patterns such that the exposed ocular tissue is ablated or excised through the process of optical breakdown or photodisruption.

### BRIEF SUMMARY OF THE INVENTION

The present invention is defined by the appended claims. Described herein is a system for marking corneal tissue in a transplant procedure. In the system, a surgical laser emits a pulsed laser beam which is directed into the cornea by a focusing assembly. An interface provides a plurality of incision patterns for selection of a sidecut pattern and an a quantity of crosscut incisions. Alternatively, the interface may provide a plurality of incision patterns, at least one of which is pre-configured with crosscuts. The selected sidecut pattern and the quantity of crosscut incisions are received by a controller which employs the focusing assembly to move the focal point of the pulsed laser beam and incise corneal tissue according to the sidecut pattern. The controller uses the quantity of crosscut incisions to determine where to place crosscut incisions across the sidecut incision. Preferably, the crosscut incisions are equally spaced about the sidecut incision.

In an exemplary method, a sidecut incision is made in each of the donor cornea and the recipient cornea. One or more crosscut incisions are made across the sidecut incision in each cornea. Corneal tissue, which is at least partially bounded by the sidecut incision made in the recipient cornea, is resected from the recipient cornea. The crosscut incision made in the recipient cornea extends beyond the resected corneal tissue. Similarly, donor tissue, which is at least partially bounded by the sidecut incision made in the donor cornea, is resected from the donor cornea. The donor tissue includes part of the crosscut incision that was made in the donor cornea. After both pieces of tissue have been resected, the donor tissue is grafted into the donor cornea. Preferably, all of the incisions are made using a surgical laser.

Other optional steps may be added to the above process, either singly or in combination. In a first optional step, the portion of the crosscut incision in the donor tissue is aligned with the portion of the crosscut incision in the recipient cornea during the grafting step. A second optional step which builds upon the first involves placing a suture along the aligned crosscut incisions. In a third optional step, multiple crosscut incisions may be made in each cornea. In a fourth optional step, the combination of incisions in each cornea form an incision pattern, with the incision pattern in the recipient cornea matching the incision pattern in the donor cornea. In a fifth optional step, the crosscut incision is made at the anterior corneal surface.

Accordingly, an improved system for marking corneal tissue in a transplant procedure is disclosed. Advantages of the improvements will appear from the drawings and the description of the preferred embodiment.

### BRIEF DESCRIPTION OF THE DRAWINGS

In the drawings, wherein like reference numerals refer to similar components:

Fig. 1 is a top plan view of a cornea and the incisions made therein;

Fig. 2 is a sectional view of the cornea of Fig. 1 along the line 2-2;

Fig. 3 is a top plan view of donor tissue grafted into a recipient cornea; and

Fig. 4 is a schematic view of a system for resecting corneal tissue.

### DETAILED DESCRIPTION OF THE INVENTION

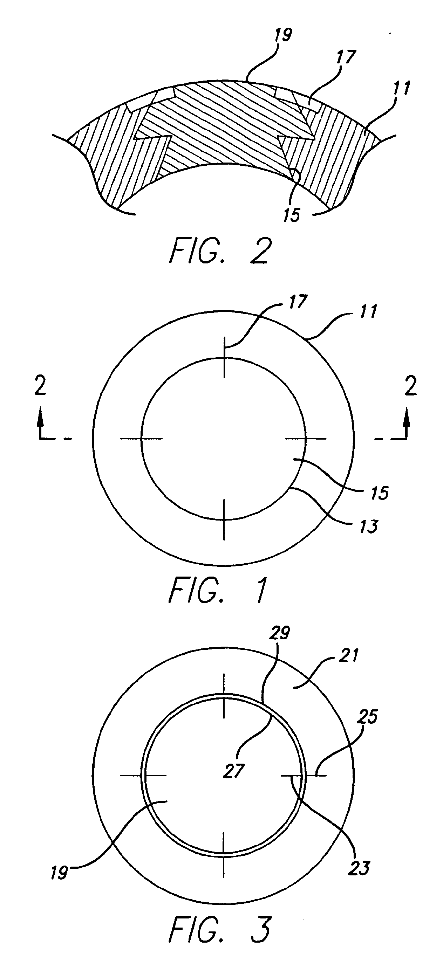

Turning in detail to the drawings, Fig. 1 illustrates the incisions made in a cornea 11 as part of a full thickness corneal transplant procedure. The same incisions are made in both the recipient cornea and the donor cornea. The sidecut incision 13 is made through the full thickness of the cornea 11. However, if a lamellar corneal transplant is performed, then the sidecut is made to the depth of the resection incision made within the stroma. In this instance, where the sidecut incision 13 is a full thickness incision, it enables resection of the corneal tissue 15 from the cornea 11. While the sidecut incision shown is a simple incision running straight from the anterior corneal surface to the posterior corneal surface, more complex sidecut incisions may also be used. Four crosscut incisions 17 are shown, and although any number of crosscut incisions may be used, at least two are preferred, with four or more being more preferred. Preferably, multiple crosscut incisions are equally spaced apart along the sidecut incision.

Fig. 2 shows the depth of the crosscuts 17 as compared to the full thickness of the cornea 11. These crosscuts 17 are located at the anterior corneal surface 19 and are deep enough so that the surgeon can easily locate them when performing the graft. The actual depth of the crosscuts 17 may therefore vary depending upon the particular preferences of the attending surgeon. The length of the crosscuts 17 also depends upon the preferences of the attending surgeon. The crosscuts 17 extend on either side of the sidecut 15 so that the surgeon can easily locate them when performing the graft. Optionally, the crosscuts may be disposed within the stroma, below the epithelium, and need not extend to the anterior corneal surface. This is also left up to the preferences of the attending surgeon.

Fig. 3 shows donor tissue 19 grafted in place within the recipient cornea 21. As indicated above, the positioning of the partial crosscuts 23, 25 within the donor tissue 19 and the recipient tissue 21 facilitates alignment of the two tissues. The amount which these partial crosscuts 23, 25 extend beyond the sidecuts 27, 29 in each of the donor tissue and the recipient cornea, respectively, facilitates placement of sutures. Additionally, a dye or stain, such as those which are well known to skilled artisans, may be used to assist the surgeon in locating and aligning the crosscuts. One suture is preferably placed at the location of each pair of partial crosscuts 23, 25. For a given pair of crosscuts 23, 25, the suture may enter the tissue at any point along the length of each partial sidecut 23, 25. This enables the surgeon to place the sutures as near or as far from the sidecuts 27, 29 as desired.

The combination of all incisions made in each of the recipient cornea and the donor cornea form an incision pattern. It is desirable to have the incision pattern in each of the two corneas be identical and symmetrical. Identical and symmetrical incision patterns enable the donor tissue to be placed in one or more orientations within the recipient cornea and greatly facilitates alignment of the crosscuts in the donor tissue with the crosscuts in the recipient cornea. Moreover, an incision pattern which includes symmetry, especially symmetry in the crosscuts, about multiple axes will help reduce the amount of stress any single suture places on the grafted tissue. Such symmetry most commonly results from the crosscuts being placed at equal intervals about the periphery of the sidecut.

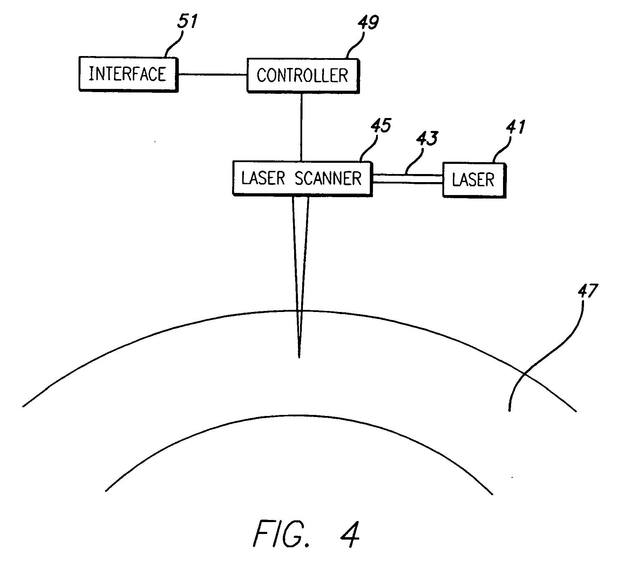

Referring to Fig. 4, a femtosecond surgical laser 41 generates a pulsed laser beam 43 and directs that beam into the focusing assembly 45, which in turn focuses the pulsed beam 43 into the cornea 47. The controller 49 is a programmable computer which precisely controls the location of the beam focal point within the cornea 47 according to parameters received from the surgeon interface 51. The interface 51 presents the surgeon with several incision patterns from which the desired sidecut pattern is selected. In addition, the interface 51 presents the surgeon with options for choosing the number of crosscut incisions. Alternatively, the interface 51 may present the surgeon with resection patterns which are pre-configured with crosscuts. The selected options are sent to the controller, and the controller 49 determines the locations of each crosscut along the sidecut pattern for purposes of controlling the focusing assembly and incising the sidecut pattern, along with the crosscuts, in the cornea.

The surgical laser may be of the type described in U.S. Patent No. 4,764,930, producing an ultra-short pulsed beam as described in one or both of U.S. Patent No. 5,984,916 and U.S. Patent No. RE37,585 to photodisrupt corneal tissues. The focusing assembly may be of the type described in U.S. Pat. App. No. 11/272,571.
Commercial laser systems capable of performing the incisions are available from IntraLase Corp. of Irvine, California.

When made with a laser, the incisions will be much less than the thickness of the needles that are typically used to place sutures, but they will still be readily visible to the surgeon (although not necessarily with the naked eye), especially if they are at the anterior corneal surface. Thus, the narrow crosscut incisions made by a laser will generally aid the surgeon in precisely locating the suture in the desired position in both the donor tissue and the recipient cornea.

The surgical laser may be used in conjunction with a contact lens (not shown) which is applied to the anterior corneal surface to deform the cornea. Deformation of the cornea in this manner provides multiple advantages which are well known to skilled artisans. For example, U.S. Patent No. 5,549,632, describes advantages gained in making laser incisions by deforming the shape of the cornea, particularly by applanation. U.S. Patent No. 6,863,667 and U.S. Pat. App. No. 11/258,399, describe patient interface devices which deform the cornea and are used to align the surgical laser with the recipient cornea for purposes of making accurate incisions.

Thus, a system for marking corneal tissue in a transplant procedure are disclosed. While embodiments of this invention have been shown and described, it will be apparent to those skilled in the art that many more modifications are possible without departing from the scope of the invention as defined by the following claims.

## Claims

1. A system for resecting corneal tissue, the system comprising:
a surgical laser (41) adapted to emit a pulsed laser beam;
a focusing assembly (45) adapted to focus the pulsed laser beam into a cornea;
an interface (51) adapted to provide a plurality of incision patterns for selection of a sidecut pattern and provide options for selection of a crosscut incision quantity; and
a controller (49) in communication with the interface, the controller being adapted to move a focal point of the pulsed laser beam within the cornea using the focusing assembly,
direct the focal point of the pulsed laser beam to make a sidecut incision in the cornea according to the sidecut pattern, wherein the corneal tissue being resected is at least partially bounded by the sidecut incision; and
make a plurality of crosscut incisions according to the crosscut incision quantity, **characterised in that** the controller is further adapted to make the crosscut incisions being incised across and on either side of the sidecut incision.

2. The system of claim 1, wherein the controller is further adapted to make the equally spaced crosscut incisions about the sidecut incision.

3. The system of claim 1, wherein the controller is further adapted to make the crosscut incisions which do not extend through the cornea.

4. The system of claim 1, wherein the controller is further adapted to make the crosscut incisions at an anterior corneal surface.

5. The system of claim 1, wherein:
the controller (49) is coupled to the focusing assembly and adapted to move the focal point of the pulsed laser beam to:
make a sidecut incision in each of the donor cornea and the recipient cornea;
make a crosscut incision across and on either side of the sidecut incision and at an anterior corneal surface in each of the donor cornea and the recipient cornea;
resect corneal tissue from the recipient cornea, the corneal tissue being at least partially bounded by the sidecut incision in the recipient cornea, wherein a first part of the crosscut incision made in the recipient cornea extends beyond the resected corneal tissue; and
resect donor tissue from the donor cornea, the donor tissue being at least partially bounded by the sidecut incision in the donor cornea, wherein a second part of the crosscut incision made in the donor cornea is in the resected donor tissue.

6. The system of claim 1, wherein:
the controller (49) is coupled to the focusing assembly and adapted to move the focal point of the pulsed laser beam to:
make a sidecut incision in each of the donor cornea and the recipient cornea;
make a plurality of crosscut incisions across and on either side of the sidecut incision in each of the donor cornea and the recipient cornea, wherein the incisions made in the recipient cornea form a first incision pattern, the incisions formed in the donor cornea form a second incision pattern, and the first incision pattern matches the second incision pattern;
resect corneal tissue from the recipient cornea, the corneal tissue being at least partially bounded by the sidecut incision in the recipient cornea, wherein a first part of each crosscut incision made in the recipient cornea extends beyond the resected corneal tissue; and
resect donor tissue from the donor cornea, the donor tissue being at least partially bounded by the sidecut incision in the donor cornea, wherein a second part of each crosscut incision made in the donor cornea is in the resected donor tissue.

7. A method practised on a dead body of resecting corneal tissue from a donor cornea comprising the steps of:
a) focusing a pulsed laser beam into a cornea;
b) selecting a sidecut pattern from a plurality of incision patterns;
c) selecting a crosscut incision quantity;
d) directing a focal point of the pulsed laser beam to make a sidecut incision in the cornea according to the selected sidecut pattern such that the corneal tissue being resected is at least partially bounded by the sidecut incision; and
e) making a plurality of crosscut incisions according to the selected crosscut incision quantity, **characterised by** the crosscut incisions being incised across and on either side of the sidecut incision.

8. The method of claim 7, wherein the crosscut incisions are equally spaced about the sidecut incision.

9. The method of claim 7, wherein the crosscut incisions do not extend through the cornea.

10. The method of claim 7, wherein the crosscut incisions are made at an anterior corneal surface.

## Patentansprüche

1. System zum Resezieren von Hornhautgewebe, wobei das System Folgendes umfasst:
einen chirurgischen Laser (41) zum Emittieren eines gepulsten Laserstrahls;
eine Fokussierungsbaugruppe (45) zum Fokussieren des gepulsten Laserstrahls in eine Hornhaut;
eine Schnittstelle (51) zum Erzeugen mehrerer Schnittmuster zum Auswählen eines Seitenschnittmusters und zum Bereitstellen von Optionen zum Auswählen eines Querschnittbetrags; und
eine Steuerung (49) in Verbindung mit der Schnittstelle, wobei die Steuerung so ausgelegt ist, dass sie einen Brennpunkt des gepulsten Laserstrahls in der Hornhaut mit der Fokussierungsbaugruppe bewegt,
Richten des Brennpunkts des gepulsten Laserstrahls zum Vornehmen eines Seitenschnitts in der Hornhaut gemäß dem Seitenschnittmuster, wobei das resezierte Hornhautgewebe wenigstens teilweise durch den Seitenschnitt begrenzt wird; und
Vornehmen mehrerer Querschnitte gemäß dem Querschnittbetrag, **dadurch gekennzeichnet, dass** die Steuerung ferner so ausgelegt ist, dass sie bewirkt, dass die Querschnitte über und auf beiden Seiten des Seitenschnitts durchgeführt werden.

2. System nach Anspruch 1, wobei die Steuerung ferner so ausgelegt ist, dass sie bewirkt, dass die Querschnitte gleichmäßig beabstandet um den Seitenschnitt durchgeführt werden.

3. System nach Anspruch 1, wobei die Steuerung ferner so ausgelegt ist, dass sie bewirkt, dass die Querschnitte so durchgeführt werden, dass sie nicht durch die Hornhaut verlaufen.

4. System nach Anspruch 1, wobei die Steuerung ferner so ausgelegt ist, dass sie bewirkt, dass die Querschnitte an einer anterioren Hornhautfläche durchgeführt werden.

5. System nach Anspruch 1, wobei:
die Steuerung (49) mit der Fokussierbaugruppe gekoppelt und so ausgelegt ist, dass sie den Brennpunkt des gepulsten Laserstrahls bewegt zum:
Vornehmen eines Seitenschnitts jeweils in der Spenderhornhaut und der Empfängerhornhaut;
Vornehmen eines Querschnitts über und auf beiden Seiten des Seitenschnitts und auf einer anterioren Hornhautfläche sowohl in der Spenderhornhaut als auch in der Empfängerhornhaut;
Resezieren des Hornhautgewebes von der Empfängerhornhaut, wobei das Hornhautgewebe wenigstens teilweise durch den Seitenschnitt in der Empfängerhornhaut begrenzt ist, wobei ein erster Teil des in der Empfängerhornhaut vorgenommenen Seitenschnitts über das resezierte Hornhautgewebe hinaus verläuft; und
Resezieren von Spendergewebe von der Spenderhornhaut, wobei das Spendergewebe wenigstens teilweise durch den Seitenschnitt in der Spenderhornhaut begrenzt wird, wobei ein zweiter Teil des in der Spenderhornhaut vorgenommenen Seitenschnitts im resezierten Spendergewebe ist.

6. System nach Anspruch 1, wobei:
die Steuerung (49) mit der Fokussierungsbaugruppe gekoppelt und so ausgelegt ist, dass sie den Brennpunkt des gepulsten Laserstrahls bewegt zum:
Vornehmen eines Seitenschnitts jeweils in der Spenderhornhaut und in der Empfängerhornhaut;
Vornehmen mehrerer Seitenschnitte über und auf beiden Seiten des Seitenschnitts jeweils in der Spenderhornhaut und in der Empfängerhornhaut, wobei die Schnitte in der Empfängerhornhaut ein erstes Schnittmuster bilden, wobei die in der Spenderhornhaut vorgenommenen Schnitte ein zweites Schnittmuster bilden und das erste Schnittmuster mit dem zweiten Schnittmuster übereinstimmt;
Resezieren des Hornhautgewebes von der Empfängerhornhaut, wobei das Hornhautgewebe wenigstens teilweise durch den Seitenschnitt in der Empfängerhornhaut begrenzt wird, wobei ein erster Teil jedes in der Empfängerhornhaut vorgenommenen Querschnitts über das resezierte Hornhautgewebe hinaus verläuft; und
Resezieren des Spendergewebes von der Spenderhornhaut, wobei das Spendergewebe wenigstens teilweise durch den Seitenschnitt in der Spenderhornhaut begrenzt wird, wobei ein zweiter Teil jedes in der Spenderhornhaut vorgenommenen Querschnitts im resezierten Spendergewebe ist.

7. Verfahren zum Resezieren von Hornhautgewebe von einer Spenderhornhaut, das an einem toten Körper ausgeführt wird und die folgenden Schritte beinhaltet:
a) Fokussieren eines gepulsten Laserstrahls in eine Hornhaut;
b) Auswählen eines Seitenschnittmusters aus mehreren Schnittmustern;
c) Auswählen eines Querschnittbetrags;
d) Richten eines Brennpunkts des gepulsten Laserstrahls, um einen Seitenschnitt in der Hornhaut gemäß dem gewählten Seitenschnittmuster vorzunehmen, so dass das resezierte Hornhautgewebe wenigstens teilweise durch den Seitenschnitt begrenzt wird; und
e) Vornehmen mehrerer Querschnitte gemäß dem gewählten Querschnittbetrag, **dadurch gekennzeichnet, dass** die Querschnitte über und auf beiden Seiten des Seitenschnitts durchgeführt werden.

8. Verfahren nach Anspruch 7, wobei die Querschnitte gleichmäßig um den Seitenschnitt herum beabstandet sind.

9. Verfahren nach Anspruch 7, wobei die Querschnitte nicht durch die Hornhaut verlaufen.

10. Verfahren nach Anspruch 7, wobei die Querschnitte an einer anterioren Hornhautoberfläche vorgenommen werden.

## Revendications

1. Système de résection d'un tissu cornéen, le système comprenant :
un laser chirurgical (41) conçu pour émettre un faisceau laser pulsé ;
un ensemble de focalisation (45) conçu pour focaliser le faisceau laser pulsé en le faisant parvenir dans une cornée ;
une interface (51) conçue pour fournir une pluralité de motifs d'incision pour la sélection d'un motif d'incision latérale et pour fournir des options pour la sélection d'un nombre d'incisions transversales ; et
un contrôleur (49) en communication avec l'interface, le contrôleur étant conçu pour
déplacer un point de convergence du faisceau laser pulsé à l'intérieur de la cornée en utilisant l'ensemble de focalisation,
diriger le point de convergence du faisceau laser pulsé pour pratiquer une incision latérale dans la cornée selon le motif d'incision latérale, le tissu cornéen résecté étant au moins en partie délimité par l'incision latérale ; et
pratiquer une pluralité d'incisions transversales selon le nombre d'incisions transversales, **caractérisé en ce que** les incisions transversales sont pratiquées en travers et de chaque côté de l'incision latérale.

2. Système selon la revendication 1, dans lequel le contrôleur est en outre conçu pour pratiquer des incisions transversales équidistantes de part et d'autre de l'incision latérale.

3. Système selon la revendication 1, dans lequel le contrôleur est en outre conçu pour pratiquer des incisions transversales qui ne s'étendent pas à travers la cornée.

4. Système selon la revendication 1, dans lequel le contrôleur est en outre conçu pour pratiquer des incisions transversales au niveau d'une surface cornéenne antérieure.

5. Système selon la revendication 1, dans lequel :
le contrôleur (49) est couplé à l'ensemble de focalisation et conçu pour déplacer le point de convergence du faisceau laser pulsé pour :
pratiquer une incision latérale dans chacune de la cornée du donneur et de la cornée du receveur ;
pratiquer une incision transversale à travers et de chaque côté de l'incision latérale et au niveau d'une surface cornéenne antérieure dans chacune de la cornée du donneur et de la cornée du receveur ;
résecter un tissu cornéen à partir de la cornée du receveur, le tissu cornéen étant au moins en partie délimité par l'incision latérale dans la cornée du receveur, une première partie de l'incision transversale pratiquée dans la cornée du receveur s'étendant au-delà du tissu cornéen résecté ; et
résecter un tissu donneur à partir de la cornée du donneur, le tissu donneur étant au moins en partie délimité par l'incision latérale dans la cornée du donneur, une deuxième partie de l'incision transversale pratiquée dans la cornée du donneur se trouvant dans le tissu donneur résecté.

6. Système selon la revendication 1, dans lequel :
le contrôleur (49) est couplé à l'ensemble de focalisation et conçu pour déplacer le point de convergence du faisceau laser pulsé pour :
pratiquer une incision latérale dans chacune de la cornée du donneur et de la cornée du receveur ;
pratiquer une pluralité d'incisions transversales à travers et de chaque côté de l'incision latérale dans chacune de la cornée du donneur et de la cornée du receveur, les incisions pratiquées dans la cornée du receveur formant un premier motif d'incision, les incisions pratiquées dans la cornée du donneur formant un deuxième motif d'incision, et le premier motif d'incision correspondant au deuxième motif d'incision ;
résecter un tissu cornéen à partir de la cornée du receveur, le tissu cornéen étant au moins en partie délimité par l'incision latérale dans la cornée du receveur, une première partie de chaque incision transversale pratiquée dans la cornée du receveur s'étendant au-delà du tissu cornéen résecté ; et
résecter un tissu donneur à partir de la cornée du donneur, le tissu donneur étant au moins en partie délimité par l'incision latérale dans la cornée du donneur, une deuxième partie de chaque incision transversale pratiquée dans la cornée du donneur se trouvant dans le tissu donneur résecté.

7. Procédé de résection d'un tissu cornéen à partir de la cornée d'un donneur mis en oeuvre sur un cadavre, consistant à :
a) focaliser un faisceau laser pulsé en le faisant parvenir dans une cornée ;
b) sélectionner un motif d'incision latérale à partir d'une pluralité de motifs d'incision ;
c) sélectionner un nombre d'incisions transversales ;
d) diriger un point de convergence du faisceau laser pulsé pour pratiquer une incision latérale dans la cornée selon le motif d'incision latérale sélectionné de telle sorte que le tissu cornéen résecté soit au moins en partie délimité par l'incision latérale ; et
e) pratiquer une pluralité d'incisions transversales selon le nombre d'incisions transversales sélectionné, **caractérisé en ce que** les incisions transversales sont pratiquées en travers et de chaque côté de l'incision latérale.

8. Procédé selon la revendication 7, dans lequel les incisions transversales sont équidistantes de part et d'autre de l'incision latérale.

9. Procédé selon la revendication 7, dans lequel les incisions transversales ne s'étendent pas à travers la cornée.

10. Procédé selon la revendication 7, dans lequel les incisions transversales sont pratiquées au niveau d'une surface cornéenne antérieure,
